# EUROPEAN PATENT SPECIFICATION

(11) **EP 3 614 983 B1**
(45) Date of publication and mention of the grant of the patent: **06.09.2023**
(21) Application number: 18724485.0
(22) Date of filing: 25.04.2018
(51) Int. Cl.: A61F 9/04, A61F 11/14

(54) **A DEVICE WEARABLE ON THE HEAD OF A USER**
AM KOPF EINES BENUTZERS TRAGBARE VORRICHTUNG
DISPOSITIF POUVANT SE PORTER SUR LA TÊTE D'UN UTILISATEUR

(30) Priority: 25.04.2017 GB 201706551
(43) Date of publication of application: 04.03.2020
(73) Proprietor: Rayne, Damian, London W11 1LR (GB)
(72) Inventor: Rayne, Damian, London W11 1LR (GB)
(74) Representative: Kilburn & Strode LLP
(86) International application number: PCT/EP2018/060618
(87) International publication number: WO 2018/197571

(56) References cited:
- WO-A2-2011/137463
- DE-U1- 8 813 099
- US-A- 6 088 836
- US-A1- 2009 255 026
- US-A1- 2012 160 240
- US-B2- 9 572 718

## Description

The present invention relates to a sleep facilitating device wearable on the head of a user and is defined in the appended claims. Disclosed is a sleep facilitating device, a personal head mask for use while sleeping or for the induction of sleep or as a personal eye mask for the same.

Many people find it difficult to fall asleep, especially in unfamiliar environments or when disturbed by sensory stimuli such as noise or unwanted light. There exist known devices for facilitating sleep which attempt to reduce or eliminate one or more sensory stimuli.

One such type of device is an eye mask, such as a blindfold, which is worn over a wearer's eyes to reduce unwanted external light from disturbing the wearer and preventing the wearer from falling asleep or causing them to awaken. A disadvantage of this type of device is that they tend to allow light to penetrate through the outer edge and the upper section of the bridge of the nose as, although contoured versions exist, they do not closely follow the contour of the wearer's face in use such that gaps exist between the blindfold and the wearer's face through which light can leak. Furthermore, existing designs use elasticated head straps and are often limited by size or simply do not allow enough pressure to hold the material of the blindfold in place and therefore the blindfold may slip out of position during use, rendering it ineffective. Eye masks generally also have straps and other components which are easy to tear and fray, especially if travel is involved, and so deteriorate quickly.

Other known devices attempt to attenuate external sound with the aim of reducing the likelihood of external noise disturbing the wearer's sleep. An example of such a device is a pair of earplugs. Earplugs suffer the disadvantage that they have a tendency to fall out during sleep, especially if the wearer sleeps on the side of their head, rendering them useless and difficult to find during the darkness and semi-consciousness of the early hours. Furthermore, earplugs decay over time, are difficult to clean and there is an additional hygiene issue associated with keeping loose earplug, often lost in bedclothes.

WO 2011/137463 A2 discloses a travel aid.

DE 88 13 099 U1 discloses a sleeping mask.

US 6 088 836 A discloses an audio/visual sensory inhibitor.

US 2012/160240 A1 discloses a dilated nasal sleep mask.

Prior art document US2009255026 A1 discloses a sleeping mask in the form of an eye shade with a conformable nose piece adapted to allow the nose portion of the eye shade to be conformed to a user's nose to substantially prevent light from passing between a user's face and the eye shade.

There is therefore a need for an improved device wearable around the head of a wearer, and in particular for an improved sleep facilitating device, or an improved personal head mask or personal eye mask, which is more effective at reducing external sensory stimuli and is more comfortable to wear, and which standardises the sleeping environment regardless of the external environment.

The present invention aims to alleviate, at least to a certain extent, the problems and/or address at least to a certain extent the difficulties associated with the prior art. For example, the present invention aims to standardise sleep by offering the user consistent protection from light and sound, irrespective of their external environment.

According to an aspect of the present invention, there is provided a sleep facilitating device wearable on the head of a wearer, the device comprising;
a head band configured to be arranged around the head of the wearer;
light-reducing means configured to reduce external light from reaching the eyes of the wearer;
the light-reducing means comprising one or more eye portions configured to be arranged over the eyes of the wearer to reduce the amount of external light reaching the eyes of the wearer; wherein the light-reducing means further comprises a nose bridge portion configured to Z receive the nose of the wearer, the nose bridge portion comprising an elasticated material, different to the material of the head band, the material being such that, when the nose bridge portion is arranged over the nose of the wearer, the elastic material is caused to stretch transversely across the width of the nose of the wearer and thereby to contract longitudinally such that the amount of external light reaching the eyes of the wearer is reduced. The longitudinal contraction of the material causes the nose bridge portion more closely conforms to the contours of the face of the wearer, thereby further reducing external light from reaching the eyes of the wearer. When the nose bridge portion is stretched transversely across the bridge of the nose of the wearer, it also substantially contracts in a perpendicular direction. This property is similar to the poisson's ratio of a material and thus materials with a high poisson's ratio could be used in the present invention. The contraction of the material causes it to compress against the skin of the user and thereby conform and tightly fit around the user's nose and the area of skin flanking each side of the nose, to thereby reduce the amount of light which is capable of seeping into the device.

Optionally, the device comprises a neck support portion configured to engage the nape of the neck of the wearer, the neck support portion comprising elastic material configured such that, when the device is arranged over the head of the wearer, the elastic material of the neck support portion is caused to stretch transversely and thereby to contract longitudinally such that the neck support portion is caused to conform to the contours of the nape of the neck of the wearer, thereby enabling the device to more securely fit to the head of the wearer. Such a support portion enables the device to be more securely held in place while in use. Optionally the support portion may be a neck support portion.

Optional features of the present invention will now be described.

Optionally, the elastic material of the nose bridge portion comprises longitudinal ribs.

Longitudinal ribs, such as those arranged parallel to each other, for example in a corrugated configuration, provide that the elastic material is able to stretch laterally to a greater extent, thereby providing a "stretchier" material, or one having greater strain (i.e. one requiring less force to provide the same extension, or the same force to provide greater extension).

Optionally, the elastic material of the nose bridge portion comprises elastic fibres.

Elastic fibres may optionally be interwoven into the fabric of the material. Elastic fibres provide for an elastic material and are particularly advantageous due to their high elasticity as well as being inexpensive.

Optionally, the nose bridge portion comprises a fleece or synthetic fur material. Fleece or synthetic fur materials provide greater levels of comfort to the wearer and additionally provide improved ability to block or reduce light from entering under the device as the base layer of the material does not have to contact the skin for light to be blocked or reduced as light may be blocked or reduced by the long fibres of the material, thus covering gaps between the device and the skin of the wearer which might otherwise exist. The nose bridge portion may instead comprise mesh material, providing improved breathability.

Optionally, the device is collapsible to a collapsed configuration. Optionally, the device, or a component thereof such as the eye portions, may be one of collapsible, crushable, scrunched, folded or the like to a collapsed, crushed, scrunched, or folded configuration respectively.

Optionally, the device further comprises sound-attenuating means configured to attenuate external sound deliverable to the ears of a wearer. The sound-attenuating means may substantially or entirely eliminate external sound.

Optionally, the sound-attenuating means comprises ear cups configured to be arranged over the ears of the wearer.

Optionally, the ear cups are configured to sit around the ears of the wearer. As such, the ear cups may be configured so as to not contact the ears of the wearer in use.

Optionally, the sound-attenuating means comprises foam. Foam is particularly advantageous as it is soft and therefore has a high degree of comfort, while also being collapsible to a collapsed configuration. Optionally, the foam may be or may comprise memory foam, for example viscoelastic polyurethane foam or low-resilience polyurethane foam.

Optionally, the sound-attenuating means are elastically crushable to a collapsed configuration. The sound-attenuating means may therefore be able to be stored in a more compact configuration, for example while they are not in use.

Optionally, the ear cups comprise a fleece material. For example, the ear cups may be covered with a fleece material. Fleece materials are particularly comfortable and provide sound attenuation, for example they may reduce wind noise.

Optionally, the device is configured to be elastically fitted around the head of the wearer.

For example, the device may optionally comprise an elasticated band or strap. Thus, the device may be more easily fitted.

Optionally, the device comprises elastic material such that the device may be stretched around the head of the wearer or provide compression around the head of the wearer when in use. The compression provided by an elastic material enables the device to remain in position more resiliently and/or securely.

The device comprises a head band configured to be arrangeable around the head of a wearer. For example, the head band may be configured to be wrapped substantially or entirely around the head of the wearer. The device may comprise affixing means for affixing, or fastening means for fastening, the head band in the wrapped configuration. A head band provides a convenient means of securing the device to the head of a wearer.

Optionally, the material of the head band comprises microfibre cloth and/or mesh material.

Optionally, the material of the head band comprises elastane.

The material of the nose bridge portion is different from the material of the head band.

Optionally, a foam insert is provided in the head band.

Optionally, the foam insert comprises the one or more eye portions and the ear cups integrally formed therein. Thus, the eye portions and the ear cups may optionally be provided as a single component.

Optionally, the head band is configured to be wrapped around the head of the wearer.

Optionally, the head band comprises a distal transverse end comprising hooks of a hook and loop fastening system such that the device may be wrapped and fastened around the head of the wearer by engagement of the hooks with a portion of the material of the device or head band. The affixing means may comprise the hooks. For example, the hooks may engage with the fibres of the material of the band. As such, the circumference of the band may be selected or adjusted, and a variety of head sizes may be accommodated, by engaging the distal end of the band along the required distance of the band.

Optionally, a proximal end of the head band comprises complimentary loops for engagement with the hooks of the hook and loop fastening system. Thus, the proximal transverse end and distal transverse end of the band may be affixed together by bringing the hooks of distal transverse end into contact with the loops of the proximal transverse end of the band. Thus, a quick and easy to use fastening means may be provided.

Optionally, the device is configured such that, when the band is wrapped around the head of the wearer, at least one of the distal and the proximal end are arranged over the face of the wearer. Thus, as a user is less likely to sleep face-down, the end of the band will not usually be compressed against the head of the user during sleep, thereby providing a more comfortable device and therefore improving the degree to which the device facilitates sleep.

Optionally, the device comprises a head support portion comprising elastic material configured such that, when the device is arranged over the head of the wearer, the elastic material of the head support portion is caused to stretch transversely and thereby to contract longitudinally such that the head support portion more closely conforms to the contours of the head of the wearer, thereby enabling the device to more securely fit to the head of the wearer. Such a head support portion enables the device to be more securely held in place while in use.

Optionally, the elastic material of the head support portion and/or the elastic material of the neck support portion is configured to provide compression when it is stretched transversely when the device is in use. Such a compression force more securely holds the device in place while in use.

Optionally, the neck support portion is made of the same material as the nose bridge portion.

Optionally, the device comprises a mesh material arranged such that the mesh material is provided at the rear of the head of the wearer in use. The mesh material may provide for a lighter device and may be breathable, thereby providing greater comfort.

Optionally, the nose bridge portion comprises mesh material.

Optionally, the sound attenuating means comprises synthetic fur material.

Optionally, the device is a head mask.

Optionally, the device is an eye mask.

The present invention may be carried out in various ways and a preferred embodiment of a device in accordance with the invention will now be described by way of example with reference to the accompanying drawings, in which:
Fig. 1 is a plan view of a preferred embodiment of a device according to a first embodiment of the present invention in which the device is unfolded and laid flat, showing the outer face or layer.
Fig. 2 is a plan view of the embodiment of Fig. 1 in which the device is unfolded and laid flat, showing the inner face or layer.
Fig. 3 is a front partial cross-sectional view of the embodiment of Fig. 1 when worn by a user, shown in ghost, the partial cross-sectional view showing the ear cups arranged around the ears of the user.
Fig. 4 is a cross-sectional view of the sound-attenuating means of the embodiment of Fig. 1, which in this embodiment comprises ear cups.
Fig. 5 is a cross-sectional view of the eye portion, light-reducing means and nose bridge portion of the embodiment of Fig. 1.
Fig. 6 is a plan view of the front (i.e. outer surface) of the insert of the embodiment of Fig. 1 showing the light-reducing means and the sound-attenuating means.
Fig. 7 is an underside view of the insert of Fig. 6 (i.e. from below).
Fig. 8 is a top view of the insert of Fig. 6 (i.e. from above).
Fig. 9 is a plan view of the rear (i.e. inner surface) of the insert of Fig. 6.
Fig. 10 is a front view of the outside of a preferred embodiment of a device according to a second embodiment of the present invention.
Fig. 11 is a front view of the inside surface of the embodiment of Fig. 10.
Fig. 12 is a side view of the outside surface of the embodiment of Fig. 10.
Fig. 13 is a rear view of the outside surface of the embodiment of Fig. 10.
Fig. 14 is a front view of the outside surface of a device useful in the understanding of the present invention.
Fig. 15 is a front view of the inside surface of the embodiment of Fig. 14.
Fig. 16 is a side view of the outside surface of the embodiment of Fig. 14.
Fig. 17 is a rear view of the outside surface of the embodiment of Fig. 14.
Fig. 18 is a front view of an alternative insert of the embodiment of Fig. 1 showing the integrally formed light-reducing means and the sound-attenuating means.
Fig. 19 is an underside view of the insert of Fig. 18.
Fig. 20 is a rear view of the insert of Fig. 18.

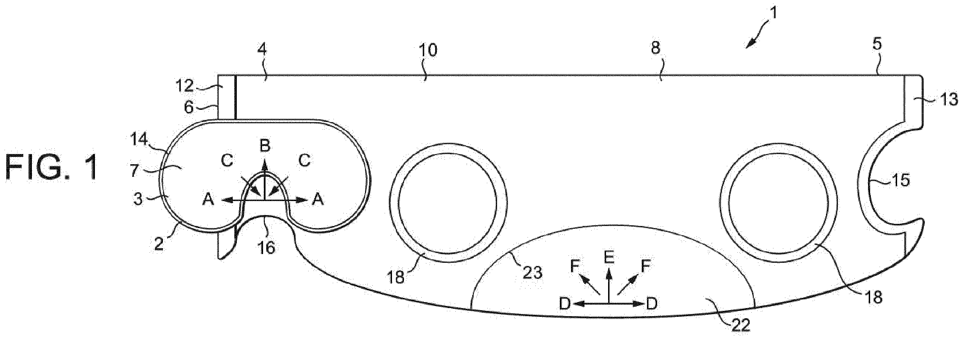

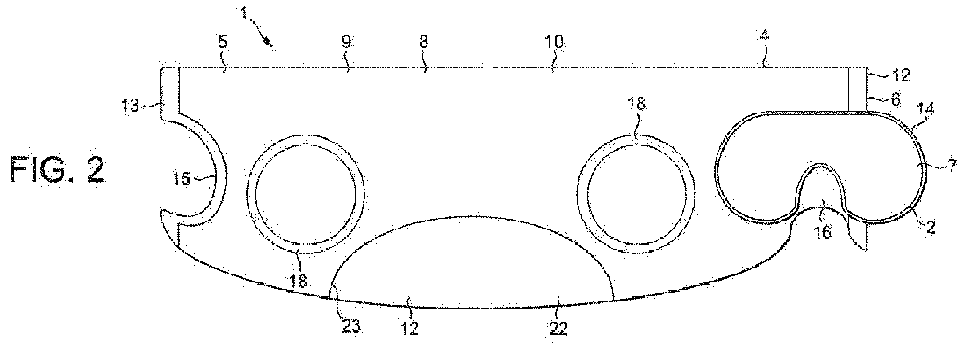

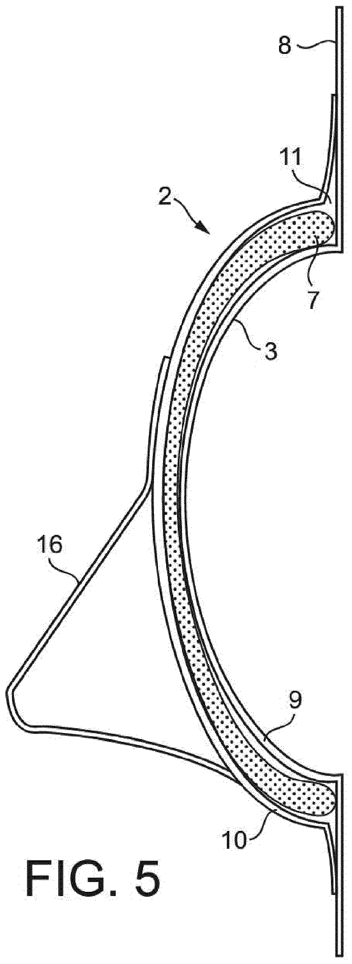

A device 1 according to a first preferred embodiment of the present invention is shown in Fig. 1. The inner surface of the device 1 is shown in Fig. 2. The device 1 is configured to be worn around the head of a user and comprises a light-reducing means 2 for preventing light from reaching the eyes of the wearer when in use. The device 1 is formed as an elongate band, strip or wrap 8 configured to be wrapped around the head of the wearer, having a first end 4 and a second end 5. Although this embodiment is formed as a band 8, and as such leaves the crown of the head uncovered, other embodiments are envisaged which may be pulled over the top of the head of the wearer, for example a hat or other such configurations, and so which cover the crown of the head of the wearer. The band 8 comprises an inner layer 9 (shown in Fig. 2), configured to contact the skin of the wearer in use, and an outer layer 10, generally opposed to the inner layer 9. The inner layer 9 and outer layer 10, or at least a portion thereof, may be fastened together, for example they may be stitched together, for example around a periphery of the device 1 or band 8, thereby defining an interval volume, envelope or pocket 11 therebetween, as can be seen in Fig. 5.

The light-reducing means 2 comprises an eye portion 3 configured to be arranged over the eyes of the wearer in use. In the embodiment shown, the eye portion 3 is provided at or generally towards the first end 4 of the band 8. The eye portion 3 may comprise any suitable means for blocking out, or reducing at least to a partial extent, light for one or both eyes, for example it may comprise an opaque layer in the form of a blindfold or one or more eye patches. In this embodiment, the eye portion comprises an insert 7, optionally made from foam and preferably made from memory foam, which is provided between the inner layer 9 and the outer layer 10 of the band and within the internal pocket 11. The foam may be polyurethane foam.

The band 8 comprises a fastening means 6 for fastening the first 4 and second ends 5 of the band 8 together for securely fitting the device 1 to the head of a wearer. The fastening means 6 optionally comprises a hook and loop fastening system, wherein one of the first 4 and second ends 5 comprises the hook portion of the hook and look fastening system, which in the example shown is provided as strip 12 which extends across the width of the band 8. In some embodiments the outer layer 10 of the band 8 is provided with the loop portion of the hook and loop fastening system or is otherwise configured, such as being constructed from a suitable material, such that the strip 12 may engage with the band various distances from the opposing end, thereby providing a device of adjustable size. For example, the loop portion may extend from the opposing end a third, quarter, or half way along the length of the band 8 or the entire outer layer 10 of the band 8 may be made from material suitable for engaging with the hooks of the hook and loop fastening means 6. Thus, the band may enable easy adjustment for accommodating various sizes of heads. In the embodiment shown, the fastening means 6 comprises a second strip 13, at an opposing end to the hook strip 12 and also extending generally across the width of the device 1, which comprises the loop portion such that the user may fasten the device by bringing the hook strip 12 and the loop strip 13 together and into contact with each other.

Whichever of the hook strip 12 or the loop strip 13 is provided at the end of the device 1 which comprises the eye portion 3, this strip 12, 13 may extend along the periphery 14 of the eye portion such that the strip generally follows the contour of the first end 4. A cut-out 15 may be provided at the second end 5, corresponding to the shape of the strip 12, 13 provided at the opposing end, for receiving said strip 12, 13.

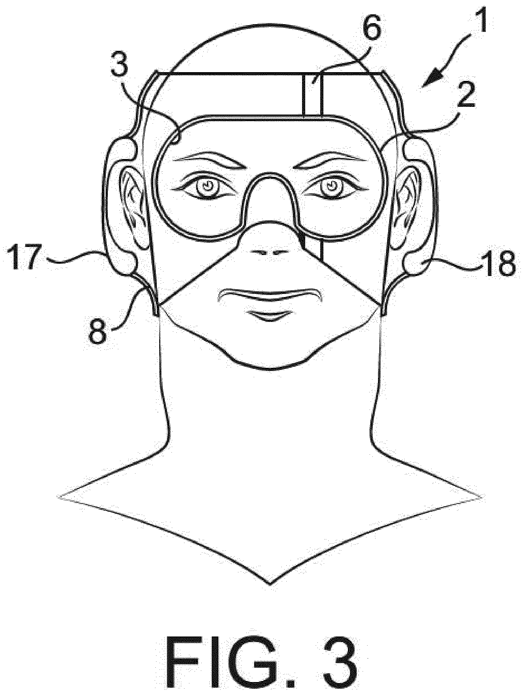

In the example shown strip 12, 13 is arranged such that it is provided generally over the face of the wearer when in use (as can be seen in Fig. 3). Advantageously, this improves comfort as the wearer is less inclined to rest their head against this portion of the device 1.

A nose bridge portion 16 is provided generally below the eye portion 3 and is arranged such that it engages with the bridge of the nose of the wearer when in use. The nose bridge portion 16 comprises a material distinct from the material of the band 8. Both, or either of, the band and the nose portion may comprise mesh material, such as elasticated mesh material. The nose bridge portion 16 comprises an elastic material which is configured such that, when the device 1 is worn, the bridge of the nose of the wearer causes the elastic material to stretch transversely (that is in the direction of arrow A of Fig. 1, i.e. in the direction from one of the first end 4 or second end 5 to the other), thereby causing the elastic material to contract substantially longitudinally (i.e. along arrow B) and causing the elastic material to move inwards, as indicated by arrow C, towards the sides of the nose of the wearer, thereby causing the nose bridge portion 16 to more closely conform to the contours of the face of the wearer. In the embodiment shown, and in the second embodiment also, the nose bridge portion is shaped so as to receive a nose of a user, for example it may optionally be substantially triangular in shape and/or contoured. By closing the gaps between the device 1 and the face of the wearer, this has the effect of reducing external light seepage which occurs in existing blindfolds and other such devices. The remainder of the band 8 surrounding the wearer's face may is not made of the same elastic material and so does not behave in the same way.

Sound-attenuating means 17 are also optionally provided for reducing the noise deliverable to the ears of the wearer. The sound-attenuating means 17 in this embodiment comprises ear cups 18 which are provided along the band 8 such that they are located over the ears of the wearer when in use. The ear cups 18 are each formed as a concave dome 19 configured to be arranged over an ear of the wearer such that the peripheral edge 20 of the dome 19 contacts the portion of the head of the wearer surrounding their ears. In this embodiment, the ear cups are configured such that they do not contact the ears directly (as may be seen in Fig. 3), although embodiments in which they are configured such that they do contact at least a portion of the ear are also envisaged (for example the concave hollow of the dome 19 may not be deep enough to accommodate or receive the ear without contacting or compressing the ear to at least a partial extent).

The ear cups 18 may optionally be made from a crushable or compressible material, such as foam (preferably memory foam), so as to provide cushioning to the wearer when the wearer rests their head against them and may be configured to direct the weight of the wearer's head when the wearer rests against them to their skull, rather than compressing their ears, thereby improving comfort. A crushable or compressible ear cup 18 also provides for a more portable or stowable device 1.

The band 8 may also optionally comprise a support portion 22 for supporting the device 1 more securely on the head of the wearer. The support portion 22 may optionally be configured to be arranged towards the rear of the head of the wearer in use and may optionally be configured to provide a compressive force to the nape of the neck of the wearer and/or lower rear of the head of the wearer. The support portion 22 may therefore be referred to as a neck support potion 22 when it is configured to engage the nape of the neck. The support portion 22 comprises elastic material, which may be the same as that of the nose bridge portion, which is configured to contract substantially longitudinally (i.e. along arrow E) when stretched transversely (i.e. along arrow D), thereby providing a compressive force (as indicated by arrows F) to the neck and/or head of the wearer such that the device is more securely held in place. As can be seen, in this embodiment, the support portion 22 is provided at a lower edge of the band 8 and is defined by an arcuate edge 23.

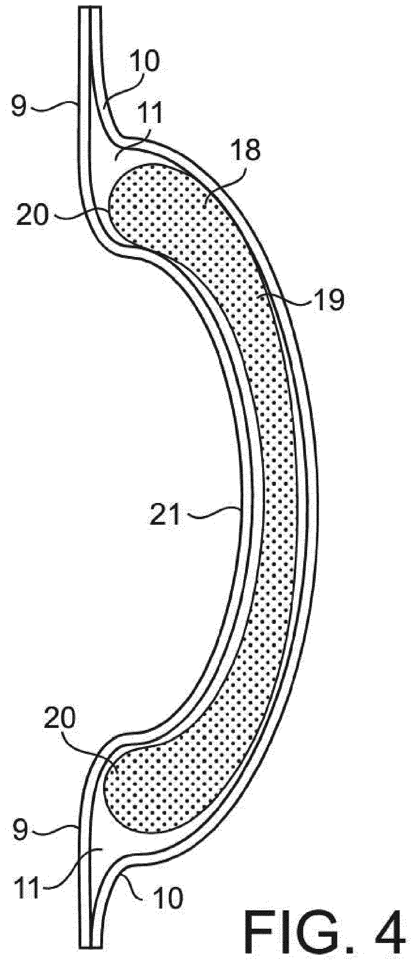

A cross-section of the portion of the band 8 comprising the ear cups 18 can be seen in Fig. 4. The foam ear cups 18 are provided in an internal pocket 11 formed between the outer layer 10 and inner layer 9 of the band 8. The inner layer 9 substantially follows the concave shape of the cup, thereby providing a hollow 21 for receiving an ear of the wearer within the concave dome-shaped ear cup 19. Although the ear cups 19 of this embodiment are concave dome-shaped, any other suitable shape for spacing the ear cup away from the ear may also be used. Embodiments are also envisaged wherein the dome-shaped ear cups 19 are not concave. As can be seen, the thickness of the ear cup 19 varies from its thickest extent at the periphery 20 of the ear cup to its thinnest extent at the peak of the dome-shape. Thus, the ear cup 18 becomes increasingly more difficult to deform as it is compressed, e.g. when a wearer rests their head against the ear cup 18. The ear cups 18 may be provided as a foam insert 7, and may be integrally formed in the insert 7, as discussed below (i.e. formed as a unitary component).

The light-reducing means 2 is shown in cross-section in Fig. 5, comprising eye portion 3. Similarly to the ear cups 18, the light-reducing means 2 comprise an insert 7 comprising at least one concave portion configured to space the device 1 away from the eyes of the wearer when in use such that the device 1, or at least the eye portion 3 thereof, does not contact the eyes of the wearer. This avoids an uncomfortable or distracting sensation of compression on the eyes which is disadvantageously provided by other known devices for facilitating sleep such as blindfolds. The insert 7 is provided within an internal pocket 11 provided between the inner layer 9 and the outer layer 10 of the band 8. The nose bridge portion 16 is also shown in cross-section. As can be seen, the thickness of the insert 7 varies from its thickest extent at the periphery 20 of the eye portion 3 to its thinnest extent at the peak of the dome-shape. Thus, the eye portion 3 becomes increasingly more difficult to deform as it is compressed, e.g. when a wearer rests their head against the eye portion 3. The eye portion 3 may be provided as a foam insert 7, and may be integrally formed in the insert 7, as discussed below. The ear cups 18 and the eye portion 3 maybe integrally formed into a single insert 7, which may be made of foam, for example memory foam (also known as viscoelastic polyurethane foam). Any of the internal pockets 11 of the present invention may be open such that the insert 7 may be readily removed or reinserted. The pockets 11 do not also need to be internal, and may instead be provided on the inner or outer surface of the device 1 and as such may be provided in an internal or external pocket or pockets 11.

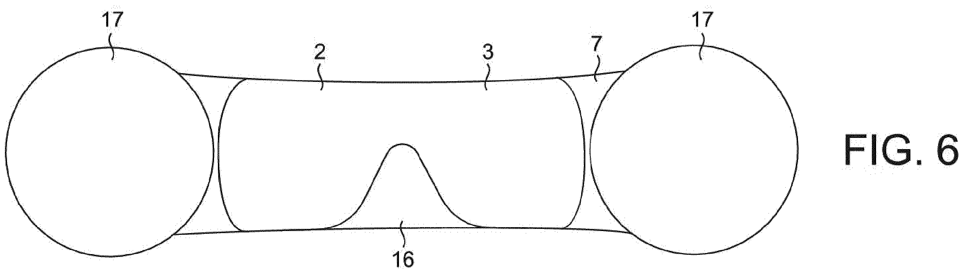

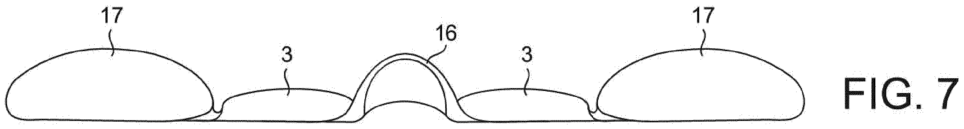

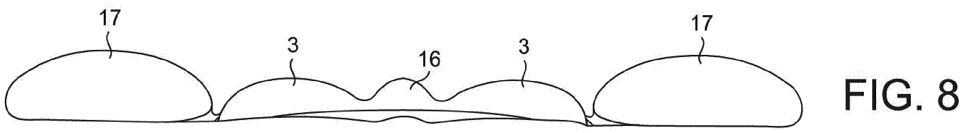

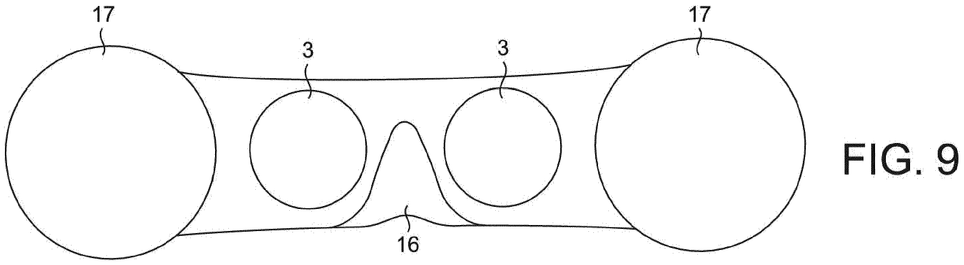

An example of a foam insert 7 into which the ear cups 18 and the eye portion 3 are integrally formed is shown in various views in Figs. 6 to 9. While embodiments are envisaged in which each ear cup 18 or eye portion 3 is received in their own internal pockets 11, this insert 7 may be provided in a single internal pocket extending from one ear cup 18 to the other, with the eye portion 3 therebetween. As can also be seen, the insert 7 of this example comprises an integral nose bridge portion 16 which is configured to be provided within, or in some examples behind, the elastic material of the nose bridge portion 16 such that the nose bridge portion 16 of the insert 7 may be deformed by the elastic material when it is stretched as discussed above.

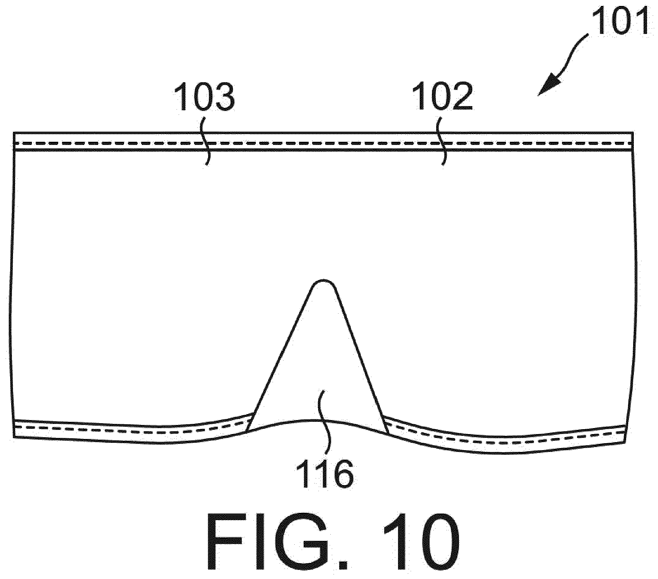

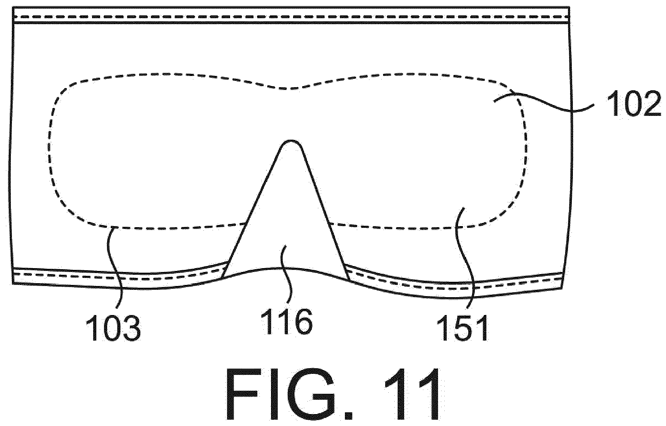

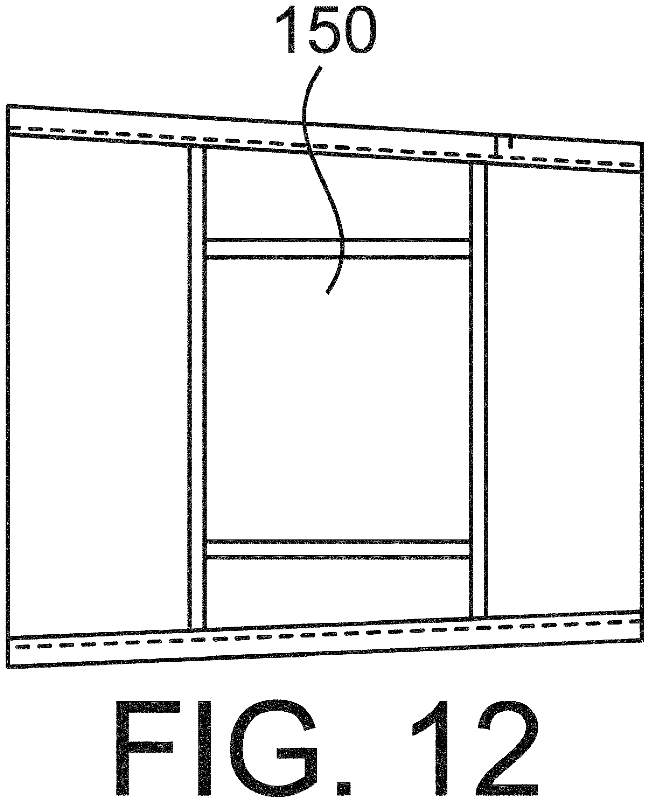

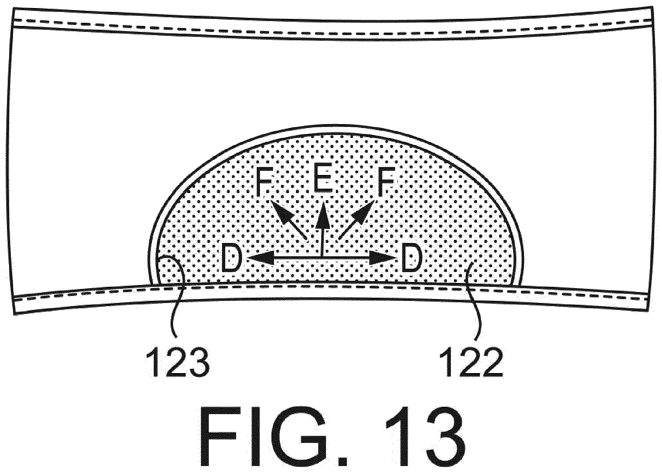

Turning now to Figs. 10 to 13, a device 101 according to a second embodiment is shown, and as such similar reference numerals have been used (increased by 100 compared to the first embodiment) to avoid redundant discussion. This embodiment is substantially the same as the first, but does not comprise the insert 7 of the eye portion 103 and does not comprise ear cups 18. The nose bridge portion 116 is made of material which behaves in the same way as that of the first embodiment such that it also more tightly conforms to the contours of the face of the wearer when it is stretched by the nose of the wearer in use, thereby providing a closer seal between the device 101 and the face of the wearer and thereby reducing external light seepage. In some embodiments, the nose bridge portion is configured to entirely conform to the shape of the face of the wearer such that there is no gap between the device 1, 101 and the face of the wearer through which light can enter.

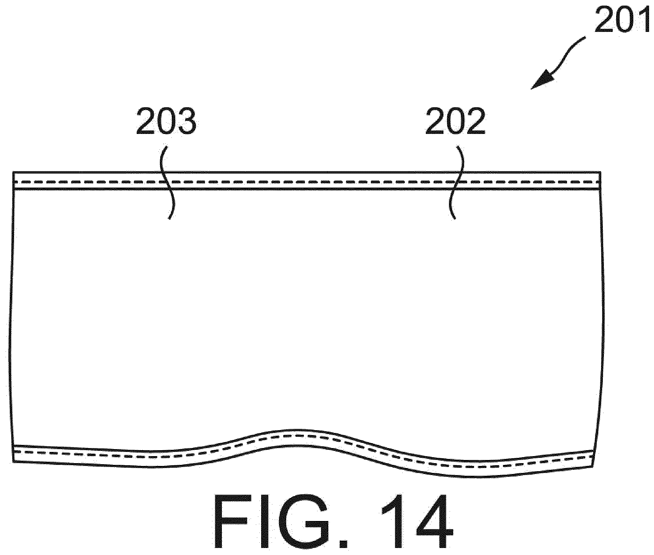

Rather than an insert 7, the eye portion 103 of this embodiment comprises an eye pad 151 affixed, e.g. stitched, to the inner surface of the band 8. Alternatively, the eye pad may be affixed to the outer surface of the band 8. Furthermore, rather than being formed as a elongate strip having ends which may be fastened together, the embodiment of Fig. 10 is formed as a sleeve which may be stretched over the head of a wearer and held in place by a support portion 122 (see Fig. 14) comprising elastic material configured to provide compression to the head of the wearer. As with the first embodiment, the support portion 122 may optionally be configured to be arranged towards the rear of the head of the wearer in use and may optionally be configured to provide a compressive force to the nape of the neck of the wearer and/or lower rear of the head of the wearer. The support portion 122 may therefore be referred to as a neck support potion 122 when it is configured to engage the nape of the neck. The support portion 122 comprises elastic material which is configured to contract substantially longitudinally (i.e. along arrow E) when stretched substantially transversely (i.e. along arrow D), thereby providing a compressive force (as indicated by arrows F) to the neck and/or head of the wearer such that the device is more securely held in place. As can be seen, in this embodiment, the support portion 122 is provided at a lower edge of the sleeve 108 and is defined by an arcuate edge 123.

The sound-attenuating means 117 of this embodiment and of other embodiments may comprise as sound-attenuating material 150, which may optionally be an integral component of the band 8, 108, which is arranged to cover the ears of the wearer, or the device 101 may alternatively not comprise any material arranged over the ears which appreciably or substantially attenuates sound and so, in these embodiments, the device 102 may be said to not comprise sound-attenuating means. An example of such material is fleece, which is particularly suited to the present invention due to its comfort. The band 8 or sleeve 108, 208 may be made of fleece, or the sound attenuating means may comprise fleece, for example on the inner 9, 109, 209 or outer 10, 110, 210 layers thereof.

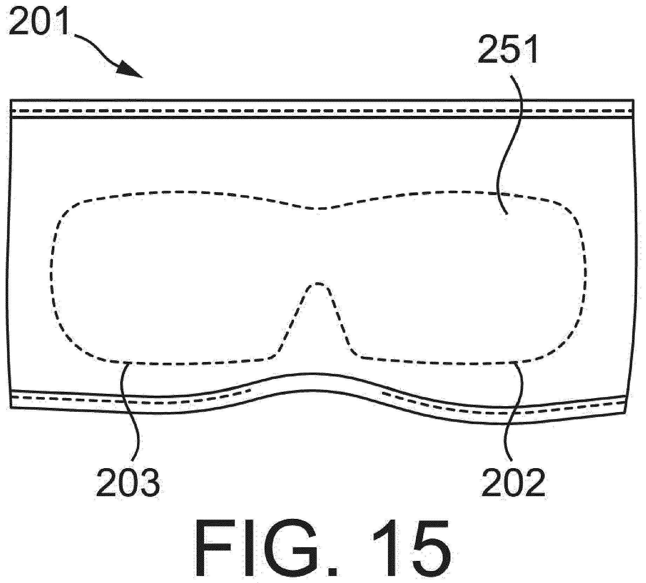

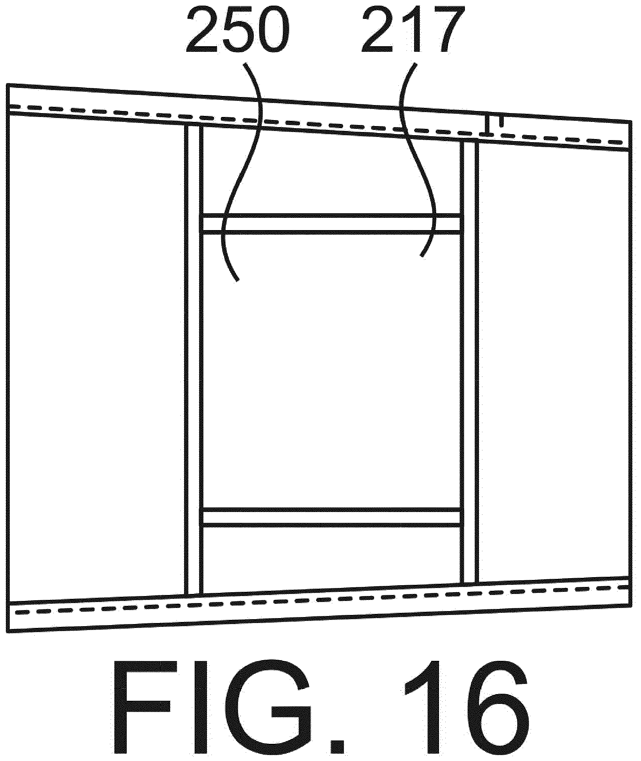

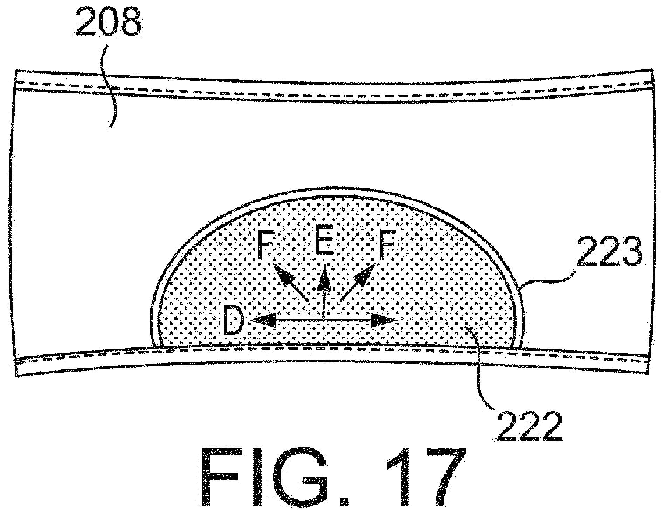

Figs. 14 to 17 show a device 201 not according to, but useful in the understanding of the present invention, and as such similar reference numerals have been used (increased by 200 compared to the first embodiment) to avoid redundant discussion. This device is substantially the same as the second embodiment, but does not comprise the elasticated nose bridge portion 116. Thus, this device is more similar to a traditional eye mask, but with a difference being the provision of a support portion 222 providing improved means for supporting the device 201 on the head of the wearer. Thus, this device is configured to be arranged around the head of a wearer and to obscure light from reaching the eyes of the wearer, as the first and second embodiments also do. As in the second embodiment, this device also includes an eye portion comprising an eye pad 251, as can be seen in Fig. 15, affixed to the inner surface of the band 108 (as indicated by the ghost line, illustrating the outline of the eye pad 251). The band 108 is also arranged to cover the ears of the wearer when in use in the same way as the second embodiment.

As with the second embodiment, this device may optionally comprise sound attenuating means and the sound attenuating means may comprise any combination of the features discussed above in relation to the sound attenuating means of the second embodiment. Similarly, this device comprises the same support portion 222 as the second embodiment and may comprise any combination of the features discussed above in relation to the support portion of the second embodiment.

The elastic material of the nose bridge portion 16, 116 and of the support portion 22, 122, 222 of the first embodiment and the second embodiment may optionally comprise longitudinal, and optionally parallel, ribs which increase the extent to which the material may stretch.

The sound attenuating means of the first and second embodiments may comprise passive or active sound isolation. For example, passive sound isolation may comprise ear plugs, which may optionally be integrally formed with the sound attenuating means, or alternatively the device may be suitable for use with ear plugs.

The material of the band 8, 108, 208 or strip may comprise elastane and, as such, be washable and breathable and have the ability to wick sweat away from the skin of the wearer. The band or strip of the first and second embodiments may also or alternatively comprise a mesh material, which may optionally be provided at the rear of the device (i.e. such that it is arranged over the rear of a wearer's head when in use). The mesh material may extend substantially from top to bottom of the device, i.e. across the width of the band or along a portion thereof. Such mesh material improves the breathability of the device and its ability to be folded, and also reduces weight. The band or strip may also comprise microfiber cloth, optionally the eye portion and/or the sound attenuating means may comprise such microfiber cloth, for example on the inner surface thereof to thereby increase comfort for the wearer. While the embodiments described above all have a nose bridge portion which may comprise, such as be made from, microfiber cloth, a mesh material could just as easily be used instead, providing improved breathability. Such a mesh material could be elasticated or otherwise elastic. The nose bridge portion and/or the sound attenuating means (such as the ear cups) could also comprise, such as be lined with, a synthetic fur material, providing improved comfort and improving the ability of the nose bridge portion to reduce light seepage and the ability of the sound attenuating means to attenuate sound.

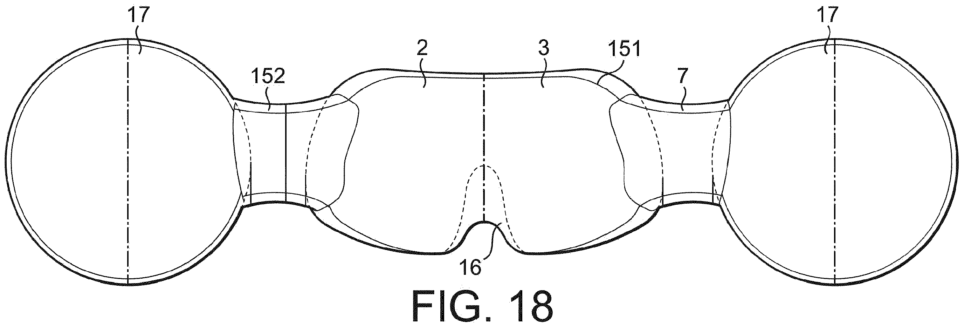

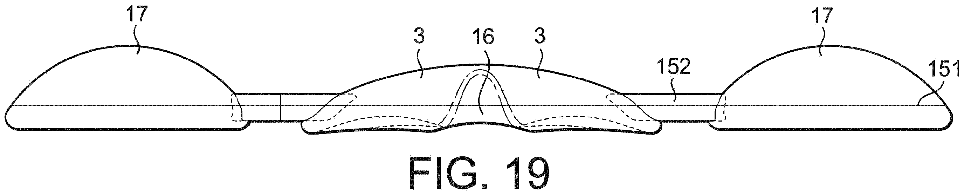

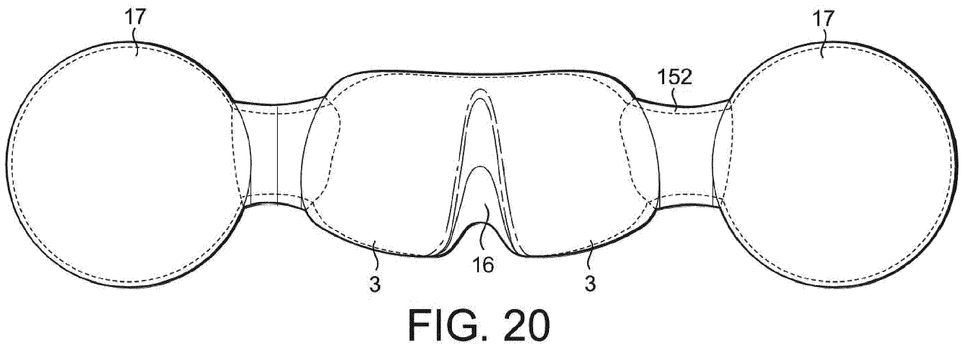

Figs. 18 to 20 show an alternative insert for use with the first embodiment. The insert 2 of Figs. 18 to 20 is substantially the same as the first insert with the only exception being that the insert is formed from two halves which are bonded (e.g. adhered) together to form the unitary insert comprising integrally formed one or more eye portions and integrally formed ear cups. The insert 7 being formed from two halves is indicated by line 151, this being the line between the two halves. In this embodiment, the two halves are a front halve and a rear half. An insert being formed from two halves in this way enables the insert to be more easily manufactured from moulds, e.g. moulds provided in a tray, and enables the insert to have contoured surfaces on both the front and rear surfaces, improving comfort. In some embodiments, the insert may be a foam insert. The foam may be an open-celled foam. The insert may comprise joining portions 152 for joining the ear cups 17 to the eye portion(s) 3, extending between each ear cup 17 and the eye portion(s) 3, and optionally the joining portions 152 may be formed, e.g. dimensioned, so as to have have a lower stiffness than the ear cups 17 and/or eye portion(s) 3. This enables the device to more readily bend and conform to the head of the user. The term "half" or "halves" in reference to the insert being formed from two "halves" should not be taken to mean that the two halves are equal in anyway, such as by weight or quantity or dimension, only that the two halves are discrete portions, which in this embodiment one of which is front-facing and the other is rear-facing. In other examples, the insert may be formed of more than two halves bonded together.

It is envisaged that the person skilled in the art may make various changes to the embodiments specifically described above without departing from the scope of the invention.

## Claims

1. A sleep facilitating device (1; 101) wearable on the head of a wearer, the device comprising;
a head band (8; 108) configured to be arranged around the head of the wearer;
light-reducing means (2) configured to reduce external light from reaching the eyes of the wearer;
the light-reducing means (2) comprising one or more eye portions (3; 103) configured to be arranged over the eyes of the wearer to reduce the amount of external light reaching the eyes of the wearer;
wherein the light-reducing means further comprises a nose bridge portion (16; 116) configured to receive the nose of the wearer, **characterised by** the nose bridge portion comprising an elastic material, different to the material of the head band, such that, when the nose bridge portion is arranged over the nose of the wearer, the elastic material is caused to stretch transversely across the width of the nose of the wearer and thereby to contract longitudinally so as to reduce the amount of external light reaching the eyes of the wearer.

2. The device (1; 101) of Claim 1, wherein the device comprises a neck support portion (22; 122)) configured to engage the nape of the neck of the wearer, the neck support portion comprising elastic material such that, when the device is arranged over the head of the wearer, the elastic material of the neck support portion is caused to stretch transversely and thereby to contract longitudinally such that the neck support portion is caused to conform to the contours of the nape of the neck of the wearer, thereby enabling the device to more securely fit to the head of the wearer.

3. The device (1; 101) of any preceding claim, wherein the elastic material of the nose bridge portion (16; 116) comprises longitudinal ribs.

4. The device (1; 101) of any preceding claim, wherein the elastic material of the nose bridge portion (16; 116) comprises elastic fibres.

5. The device (1; 101) of any preceding claim, wherein the nose bridge portion comprises (16; 116) a fleece or synthetic fur material.

6. The device (1; 101) of any preceding claim, wherein the device further comprises sound-attenuating means (17; 117) configured to attenuate external sound deliverable to the ears of a wearer, optionally wherein the sound-attenuating means comprises synthetic fur material and/or foam.

7. The device (1; 101) of Claim 6, wherein the sound-attenuating means (17; 117) comprises ear cups (18; 118), optionally comprising a fleece material, configured to be arranged over the ears of the wearer and optionally configured to sit off the ears of the wearer.

8. The device (1; 101) of any preceding claim, wherein the material of the head band comprises microfibre cloth and/or mesh material.

9. The device (1; 101) of any preceding claim, wherein the material of the head band (8; 108) comprises elastane.

10. The device (1; 101) of any preceding claim, wherein a foam insert (7) is provided in the head band (8; 108), optionally wherein the foam insert is formed of at least two halves bonded together.

11. The device (1; 101) of Claim 10, wherein the foam insert (7) comprises the one or more eye portions (3; 103) and the ear cups (7; 107) integrally formed therein.

12. The device (1; 101) of any preceding claim, wherein the head band (8; 108) is configured to be wrapped around the head of the wearer.

13. The device (1; 101) of any preceding claim, wherein the head band (8; 108) comprises a distal transverse end (4) comprising hooks of a hook and loop fastening system such that the device may be wrapped and fastened around the head of the wearer by engagement of the hooks with a portion of the material of the device or head band, optionally wherein a proximal end (5) of the head band comprises complimentary loops for engagement with the hooks of the hook and loop fastening system, optionally wherein the device is configured such that, when the band is wrapped around the head of the wearer, at least one of the distal and the proximal end are arranged over the face of the wearer.

14. The device (1; 101) of any preceding claim when dependent on Claim 2 , wherein the neck support portion (22; 122) is made of the same material as the nose bridge portion (16; 116).

15. The device (1; 101) of any preceding claim, wherein the device comprises a mesh material arranged such that the mesh material is provided at the rear of the head of the wearer in use.

## Patentansprüche

1. Den Schlaf erleichternde Vorrichtung (1; 101), die am Kopf eines Trägers tragbar ist, wobei die Vorrichtung umfasst:
ein Stirnband (8; 108), das dazu ausgelegt ist, um den Kopf des Trägers angeordnet zu werden;
ein lichtreduzierendes Mittel (2), das dazu ausgelegt ist, auf die Augen des Trägers auftreffendes externes Licht zu reduzieren;
wobei das lichtreduzierende Mittel (2) einen oder mehrere Augenabschnitte (3; 103) umfasst, die dazu ausgelegt sind, über den Augen des Trägers angeordnet zu werden, um die Menge an auf die Augen des Trägers auftreffendem Licht zu reduzieren;
wobei das lichtreduzierende Mittel ferner einen Nasenstegabschnitt (16; 116) umfasst, der dazu ausgelegt ist, die Nase des Trägers aufzunehmen, **dadurch gekennzeichnet, dass** der Nasenstegabschnitt ein elastisches Material umfasst, das sich von dem Material des Stirnbandes unterscheidet, sodass, wenn der Nasenstegabschnitt über der Nase des Trägers angeordnet ist, bewirkt wird, dass das elastische Material quer über die Breite der Nase des Trägers gedehnt wird und sich dadurch längs zusammenzieht, um die Menge an auf die Augen des Trägers auftreffendem externem Licht zu reduzieren.

2. Vorrichtung (1; 101) nach Anspruch 1, wobei die Vorrichtung einen Halsstützabschnitt (22; 122) umfasst, der dazu ausgelegt ist, den Nacken des Trägers in Eingriff zu nehmen, wobei der Halsstützabschnitt ein elastisches Material umfasst, sodass, wenn die Vorrichtung am Kopf des Trägers angeordnet wird, bewirkt wird, dass sich das elastische Material des Halsstützabschnitts quer dehnt und sich dadurch längs zusammenzieht, sodass bewirkt wird, dass sich der Halsstützabschnitt an die Konturen des Nackens des Trägers anpasst und dadurch ermöglicht, dass die Vorrichtung fester am Kopf des Trägers sitzt.

3. Vorrichtung (1; 101) nach einem vorhergehenden Anspruch, wobei das elastische Material des Nasenstegabschnitts (16; 116) Längsrippen umfasst.

4. Vorrichtung (1; 101) nach einem vorhergehenden Anspruch, wobei das elastische Material des Nasenstegabschnitts (16; 116) elastische Fasern umfasst.

5. Vorrichtung (1; 101) nach einem vorhergehenden Anspruch, wobei der Nasenbrückenabschnitt (16; 116) ein Fleece- oder synthetisches Fellmaterial umfasst.

6. Vorrichtung (1; 101) nach einem vorhergehenden Anspruch, wobei die Vorrichtung ferner ein geräuschdämpfendes Mittel (17; 117) umfasst, das dazu ausgelegt ist, externe Geräusche, die an die Ohren eines Trägers übermittelt werden können, zu dämpfen, wobei optional das geräuschdämpfende Mittel synthetisches Fellmaterial und/oder Schaum umfasst.

7. Vorrichtung (1; 101) nach Anspruch 6, wobei das geräuschdämpfende Mittel (17; 117) Ohrmuscheln (18; 118) umfasst, die optional ein Fleece-Material umfassen und dazu ausgelegt sind, über den Ohren des Trägers angeordnet zu werden und optional dazu ausgelegt sind, vor den Ohren des Trägers zu sitzen.

8. Vorrichtung (1; 101) nach einem vorhergehenden Anspruch, wobei das Material des Stirnbands Mikrofasertuch und/oder Mesh-Material umfasst.

9. Vorrichtung (1; 101) nach einem vorhergehenden Anspruch, wobei das Material des Stirnbands (8; 108) Elasthan umfasst.

10. Vorrichtung (1; 101) nach einem vorhergehenden Anspruch, wobei ein Schaumeinsatz (7) in dem Stirnband (8; 108) bereitgestellt ist, wobei optional der Schaumeinsatz aus mindestens zwei zusammengefügten Hälften gebildet ist.

11. Vorrichtung (1; 101) nach Anspruch 10, wobei der Schaumeinsatz (7) den einen oder die mehreren Augenabschnitte (3; 103) und die darin einstückig gebildeten Ohrmuscheln (7; 107) umfasst.

12. Vorrichtung (1; 101) nach einem vorhergehenden Anspruch, wobei das Stirnband (8; 108) dazu ausgelegt ist, um den Kopf des Trägers gewickelt zu werden.

13. Vorrichtung (1; 101) nach einem vorhergehenden Anspruch, wobei das Stirnband (8; 108) ein distales Querende (4) umfasst, das Haken eines Klettverschlusssystems umfasst, sodass die Vorrichtung um den Kopf des Trägers gewickelt und durch den Eingriff der Haken mit einem Abschnitt des Materials der Vorrichtung oder des Stirnbands befestigt wird, wobei optional ein proximales Ende (5) des Stirnbandes komplementäre Schleifen zum Eingriff mit den Haken des Klettverschlusssystems umfasst, wobei optional die Vorrichtung derart ausgelegt ist, dass, wenn das Band um den Kopf des Trägers gewickelt wird, mindestens eins des distalen und des proximalen Endes im Gesicht des Trägers angeordnet ist.

14. Vorrichtung (1; 101) nach einem vorhergehenden Anspruch in Abhängigkeit von Anspruch 2, wobei der Halsstützabschnitt (22; 122) aus demselben Material besteht wie der Nasenstegabschnitt (16; 116).

15. Vorrichtung (1; 101) nach einem vorhergehenden Anspruch, wobei die Vorrichtung ein Mesh-Material umfasst, das derart angeordnet ist, dass das Mesh-Material im Gebrauch auf der Rückseite des Kopfes des Trägers bereitgestellt ist.

## Revendications

1. Dispositif facilitant le sommeil (1 ; 101) pouvant être porté sur la tête d'un utilisateur, le dispositif comprenant ;
un bandeau (8 ; 108) conçu pour être agencé autour de la tête de l'utilisateur ;
des moyens de réduction de lumière (2) conçus pour réduire la lumière externe atteignant les yeux de l'utilisateur ;
les moyens de réduction de lumière (2) comprenant une ou plusieurs parties pour les yeux (3 ; 103) conçues pour être agencées sur les yeux de l'utilisateur afin de réduire la quantité de lumière externe atteignant les yeux de l'utilisateur ;
dans lequel les moyens de réduction de lumière comprennent en outre une partie pont de nez (16 ; 116) conçue pour recevoir le nez de l'utilisateur, **caractérisé par** la partie pont de nez comprenant un matériau élastique, différent du matériau du bandeau, de telle sorte que, lorsque la partie pont de nez est agencée sur le nez de l'utilisateur, le matériau élastique soit amené à s'étirer transversalement sur la largeur du nez de l'utilisateur et de ce fait à se contracter longitudinalement de manière à réduire la quantité de lumière externe atteignant les yeux de l'utilisateur.

2. Dispositif (1 ; 101) selon la revendication 1, le dispositif comprenant une partie support de cou (22 ; 122)) conçue pour entrer en prise avec la nuque de l'utilisateur, la partie support de cou comprenant un matériau élastique de telle sorte que, lorsque le dispositif est agencé au-dessus de la tête de l'utilisateur, le matériau élastique de la partie support de cou soit amené à s'étirer transversalement et, de ce fait, à se contracter longitudinalement de telle sorte que la partie support de cou soit amenée à épouser les contours de la nuque de l'utilisateur, permettant de ce fait que le dispositif s'ajuste plus solidement sur la tête de l'utilisateur.

3. Dispositif (1; 101) selon l'une quelconque revendication précédente, dans lequel le matériau élastique de la partie pont de nez (16 ; 116) comprend des nervures longitudinales.

4. Dispositif (1; 101) selon l'une quelconque revendication précédente, dans lequel le matériau élastique de la partie pont de nez (16 ; 116) comprend des fibres élastiques.

5. Dispositif (1; 101) selon l'une quelconque revendication précédente, dans lequel la partie pont de nez (16 ; 116) comprend un matériau en polaire ou en fourrure synthétique.

6. Dispositif (1; 101) selon l'une quelconque revendication précédente, le dispositif comprenant en outre des moyens d'atténuation sonore (17 ; 117) conçus pour atténuer un son externe pouvant être délivré aux oreilles d'un utilisateur, éventuellement dans lequel les moyens d'atténuation sonore comprennent un matériau en fourrure synthétique et/ou de la mousse.

7. Dispositif (1 ; 101) selon la revendication 6, dans lequel les moyens d'atténuation sonore (17 ; 117) comprennent des oreillettes (18 ; 118), comprenant éventuellement un matériau en polaire, conçues pour être agencées sur les oreilles de l'utilisateur et éventuellement conçues pour se décaler des oreilles de l'utilisateur.

8. Dispositif (1; 101) selon l'une quelconque revendication précédente, dans lequel le matériau du bandeau comprend un tissu en microfibre et/ou un matériau en maille.

9. Dispositif (1; 101) selon l'une quelconque revendication précédente, dans lequel le matériau du bandeau (8 ; 108) comprend de l'élasthanne.

10. Dispositif (1; 101) selon l'une quelconque revendication précédente, dans lequel un insert en mousse (7) est prévu dans le bandeau (8 ; 108), éventuellement dans lequel l'insert en mousse est formé d'au moins deux moitiés collées l'une à l'autre.

11. Dispositif (1 ; 101) selon la revendication 10, dans lequel l'insert en mousse (7) comprend les une ou plusieurs parties pour les yeux (3 ; 103) et les oreillettes (7 ; 107) formées d'un seul tenant à l'intérieur.

12. Dispositif (1; 101) selon une quelconque revendication précédente, dans lequel le bandeau (8 ; 108) est conçu pour être enroulé autour de la tête de l'utilisateur.

13. Dispositif (1; 101) selon l'une quelconque revendication précédente, dans lequel le bandeau (8 ; 108) comprend une extrémité transversale distale (4) comprenant des crochets d'un système de fixation à boucles et à crochets de telle sorte que le dispositif puisse être enroulé et fixé autour de la tête de l'utilisateur par entrée en prise des crochets avec une partie du matériau du dispositif ou du bandeau, éventuellement, dans lequel une extrémité proximale (5) du bandeau comprend des boucles complémentaires pour une entrée en prise avec les crochets du système de fixation à boucles et à crochets, éventuellement, dans lequel le dispositif est conçu de telle sorte que, lorsque le bandeau est enroulé autour de la tête de l'utilisateur, au moins l'une de l'extrémité distale et de l'extrémité proximale soit agencée sur le visage de l'utilisateur.

14. Dispositif (1; 101) selon l'une quelconque revendication précédente lorsqu'elle dépend de la revendication 2, dans lequel la partie support de cou (22 ; 122) est réalisée dans le même matériau que la partie pont de nez (16 ; 116).

15. Dispositif (1; 101) selon l'une quelconque revendication précédente, dans lequel le dispositif comprend un matériau en maille agencé de telle sorte que le matériau en maille soit prévu à l'arrière de la tête de l'utilisateur lors de l'utilisation.
